# EUROPEAN PATENT SPECIFICATION

(11) **EP 0 839 061 B1**
(45) Date of publication and mention of the grant of the patent: **25.06.2003**
(21) Application number: 97920698.4
(22) Date of filing: 16.04.1997
(51) Int. Cl.: A61L 9/03

(54) **MULTIPLE ELECTRIC VAPORIZER FOR RESINS**
ELEKTRISCHE MEHRFACHVERDAMPFER FÜR HARZE
VAPORISATEUR ELECTRIQUE DE RESINES POURVU D'UNE PLURALITE DE CORPS

(30) Priority: 19.04.1996 IT PD960097
(43) Date of publication of application: 06.05.1998
(73) Proprietor: D'Arienzo, Anna Maria, 35128 Padova (IT)
(72) Inventor: BEVILACQUA, Matteo, I-35128 Padova (IT); ZACCAGNA, Carlo, Alberto, I-10090 Villarbasse (IT)
(74) Representative: Modiano, Guido, Dr.-Ing.
(86) International application number: EP9701897
(87) International publication number: WO97039779

(56) References cited:
- WO-A-90/03192
- FR-A- 2 380 002
- FR-A- 2 510 410
- FR-A- 2 614 535
- GB-A- 2 062 199
- US-A- 5 178 839

## Description

### Technical Field

The present invention relates to a multiple electric vaporizer for vaporizing resins such as incense, propolis and myrrh.

### Background Art

Conventional vaporizers for insecticides or resins are adapted to heat impregnated tablets, liquid containers, or resins in various forms.

These electrically powered vaporizers are particularly simple, but indeed because of their simplicity they are not functional, since their temperature adjustment is very inaccurate. Such vaporisers are disclosed for example in FR-A-2510410 and GB-A-2062199.

Temperature is an important variable in achieving correct vaporization without causing carbonization of the product, which generates toxic compounds especially if the product is resin-based.

Resin-derived products or products having a resin-like consistency, such as incense, myrrh and propolis, conventionally have therapeutic characteristics which are enhanced when they are inhaled in vapor form and even more so if these vapors are produced by mixtures of products.

### Disclosure of the Invention

A principal aim of the present invention is to provide a multiple electric vaporizer which allows to vaporize a plurality of products simultaneously.

Within the scope of this aim, an object of the present invention is to provide a multiple electric vaporizer in which it is possible to vaporize different products at different temperatures.

Another object of the present invention is to provide an electric vaporizer which is unexpensive and easy to use.

This aim, these objects and others which will become apparent hereinafter are achieved by a multiple electric vaporizer for resins, characterized in that it comprises a supporting structure for a plurality of electrically heated hollow bodies, each whereof is adapted to accommodate an ampoule containing a product to be vaporized, said structure containing electric and electronic power supply and control means for vaporising different resin products at different temperatures, a perforated dome being located above the heating bodies and being adapted to protect them and mix the generated vapors, internal ventilation means being also provided.

### Brief Description of the Drawings

Further characteristics and advantages of the present invention will become apparent from the following detailed description of a preferred embodiment thereof, given only by way of non-limitative example and illustrated in the accompanying drawings, wherein:

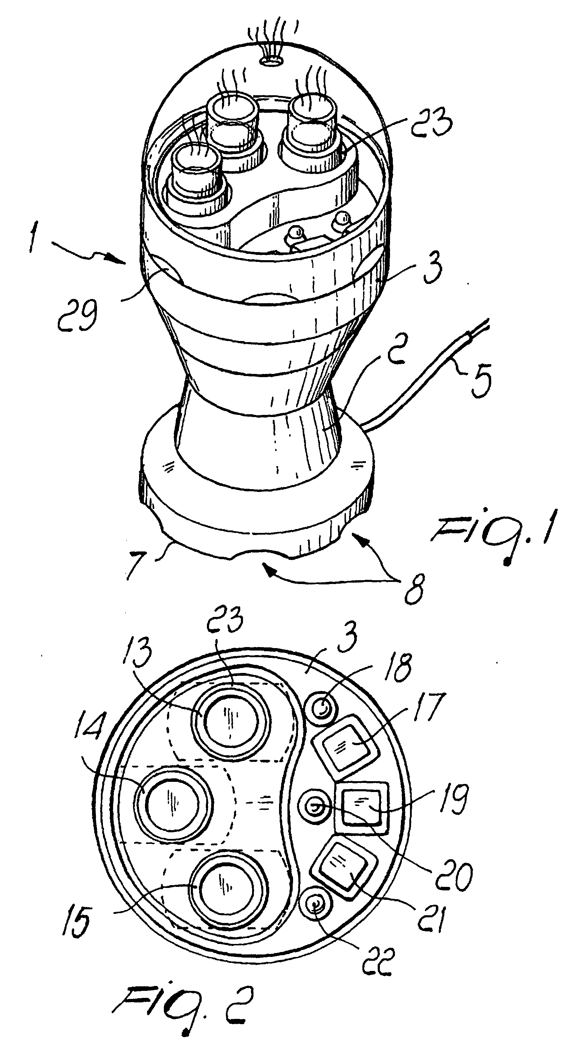

figure 1 is a perspective view of the electric vaporizer according to the present invention;
figure 2 is a top view of the electric vaporizer of figure 1;
figure 3 is a sectional view of the electric vaporizer;
figure 4 is a partial, sectional view of a different embodiment of said electric vaporizer.

### Ways of carrying out the Invention

With reference to the above figures, the electric vaporizer according to the present invention is composed of a structure generally designated by the reference numeral 1 and composed of a footing 2 and a head 3, both being hollow and preferably produced by molding plastics.

A step-down transformer, not shown, is located inside the footing, in a box-like body 4, and is supplied from the mains by means of a power cord 5.

The lower part of the footing 2 is wider and is kept raised from the resting surface 6 by a cylindrical ring 7 having a plurality of arc-shaped openings 8.

Vertical channels 9 are provided inside footing 2 and lead into an annular chamber 10 which surrounds the box-like body 4 containing the transformer.

Chamber 10 is provided, in an upward region, with passages 11 leading into an upper part of the footing 2, where a second box-like body 12 is provided which contains electrical and electronic devices for control and operation.

The footing 2 is coupled to the head 3, with which three hollow heating bodies 13, 14 and 15 made of metal are associated.

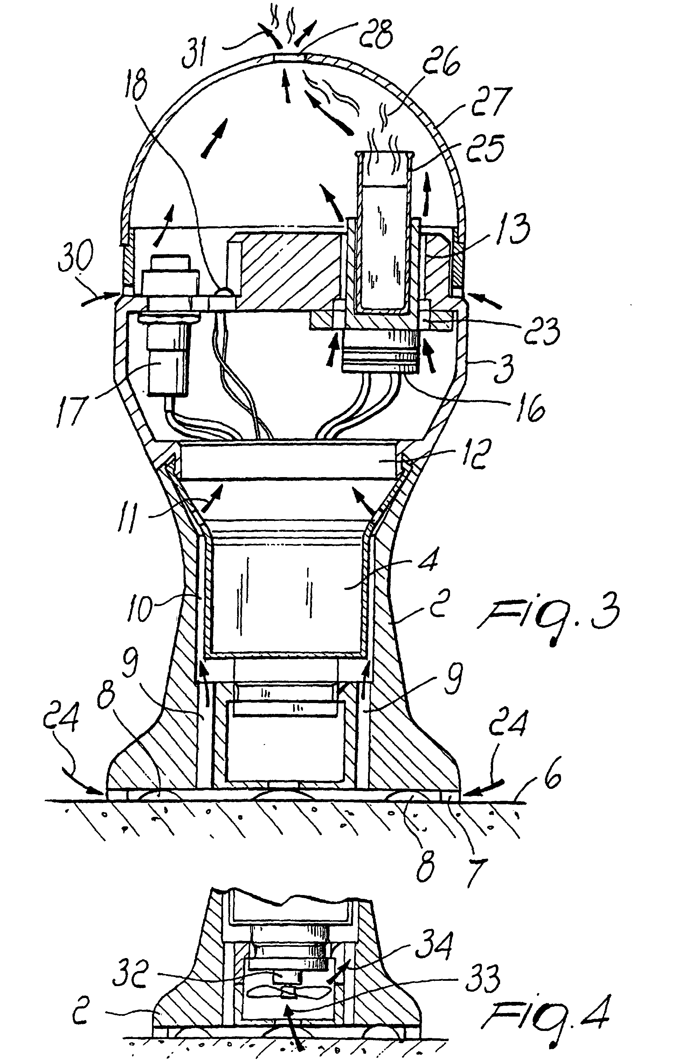

An electric resistor 16 is provided in the lower part of each one of said heating bodies, for example the one designated by the reference numeral 13 in figure 3; the resistor can be activated by a switch 17, its operation being visualized by a luminous indicator 18.

Likewise, the hollow heating body 14 is controlled by a switch 19 provided with a visual indicator 20 and the hollow heating body 15 is controlled by a switch 21 provided with a visual indicator 22.

An annular chamber for the flow of air, designated by the reference numeral 23 for the body 13, is provided around each one of said hollow bodies 13, 14 and 15.

An identical chamber is also provided around the hollow bodies 14 and 15.

All the above referenced ducts and chambers allow ambient air to enter from below, in the direction indicated by the arrows 24, and to rise through the various ducts and the various chambers, cooling the transformer and then flowing over the outer part of each hollow body in the direction indicated by the arrows shown in figure 3.

An ampoule 25 containing the resin to be vaporized is inserted in each hollow body.

Heating of the respective hollow body by means of the resistor, for example 16, causes a production of vapors 26 which first gather beneath a protective dome 27 and then flow outside through an upper hole 28.

The dome 27 is preferably made of transparent plastics and has at its lower edge, where it makes contact with the head 3, a plurality of openings 29 which allow air to enter in the direction shown by the arrow 30, the air mixing with the vapors and also flowing out of the hole 28 in the direction shown by the arrows 31.

The resistors of the three hollow heating bodies are controlled so that the temperature reaches a preset value and then remains constant.

The three hollow bodies have different operating temperatures which can in any case be preset or adjusted, depending on the resin to be vaporized.

More particularly, the electric vaporizer according to the present invention is conveniently used to vaporize propolis, incense and myrrh separately or together.

For these products, the operating temperatures are between 90° and 110° for the hollow body meant to vaporize propolis, between 90° and 120° for the hollow body meant to vaporize incense, and between 90° and 130° for the hollow body meant to vaporize myrrh.

Temperature adjustment allows to obtain a vapor mixture which is blended at pleasure.

The electronic control system is also conveniently provided with a timer system, so as to allow to preset the vaporization periods.

In order to increase the introduction of the vapors into the environment, it can be convenient to install, as shown in figure 4 in the lowermost part of the footing 2, a fan 32 which causes forced air suction in the direction shown by the arrow 33 and sends it, again with a forced action, through the channels and the circulation chambers which have already been described.

The description of the embodiment clearly shows that the electric vaporizer according to the present invention allows to simultaneously vaporize three different products at different temperatures and, if one wishes, for different times.

It is thus possible to introduce into an enclosed space a chosen amount of products, so as to utilize their therapeutic characteristics in an optimum manner.

The electric vaporizer is very simple to use and is protected in its hot parts, so that it is not dangerous.

Obviously, the components used and the shapes of the structure may be different although starting from the same inventive concept.

## Claims

1. A multiple electric vaporizer for resins,
**characterized in that** it comprises a supporting structure (2) for a plurality of electrically heated hollow bodies (13,14,15), each whereof is adapted to accommodate an ampoule (25) containing a resin product to be vaporized, said structure containing electric for vaporizing different resin products at different temperatures and electronic power supply and control means (12), a perforated dome (27) being located above the heating bodies and being adapted to protect them and mix the generated vapors.

2. A multiple electric vaporizer according to claim 1, **characterized in that** said supporting structure is constituted by a footing (2) and by a head (3), both being hollow.

3. An electric vaporizer according to claim 2, **characterized in that** a step-down transformer (4) is contained in said footing, in a chamber (10) which is externally ventilated by air streams arriving from the lower part of the footing, which is raised and has a plurality of external accesses (8).

4. An electric vaporizer according to claim 3, **characterized in that** it comprises a fan (32) contained in said footing.

5. An electric vaporizer according to claim 1, **characterized in that** said hollow bodies are metallic, with cylindrical cavities having a vertical axis, and are heated in a downward region by a resistor (16) which can be activated by means of a switch (17) and is equipped with a thermostat.

6. An electric vaporizer according to claim 1, **characterized in that** said hollow bodies are fixed to the head of said structure so as to leave a perimetric opening (29) which allows said ventilation air to flow over them externally, producing a rising flow which mixes the released vapors.

7. An electric vaporizer according to claim 1, **characterized in that** said head is closed by a detachable dome (27) for protecting and mixing the vapors, and is provided with lower openings (29) for the inflow of air and with upper holes (28) for the exit of the vapors.

8. An electric vaporizer according to claim 1, **characterized in that** timer means for adjusting the activation and deactivation of the heating resistors are provided.

9. An electric vaporizer according to claim 1, **characterized in that** the temperatures in three heating bodies are respectively between 90° and 110° Celsius for vaporizing propolis, between 90° and 120° Celsius for vaporizing incense, and between 90° and 130° Celsius for vaporizing myrrh.

## Patentansprüche

1. Elektrischer Mehrfachverdampfer für Harze, **dadurch gekennzeichnet, dass** er eine Haltekonstruktion (2) für eine Mehrzahl von elektrisch beheizten Hohlkörpern (13, 14, 15) umfasst, von denen jeder angepasst ist, um eine Ampulle (25) zu beherbergen, die ein zu verdampfendes Harzprodukt enthält, wobei die besagte Konstruktion elektrische und elektronische Stromversorgungs- und Steuereinrichtungen (12) zum Verdampfen von verschiedenen Harzprodukten bei verschiedenen Temperaturen enthält, wobei eine perforierte Haube (27) über den Heizkörpern angeordnet ist und angepasst ist, um sie zu schützen und die erzeugten Dämpfe zu vermischen.

2. Elektrischer Mehrfachverdampfer nach Anspruch 1, **dadurch gekennzeichnet, dass** die besagte Haltekonstruktion von einem Fuß (2) und von einem Kopf (3) gebildet wird, die beide hohl sind.

3. Elektrischer verdampfer nach Anspruch 2, **dadurch gekennzeichnet, dass** ein Abwärtstransformator (4) in besagtem Fuß enthalten ist, in einer Kammer (10), die durch Luftströme äußerlich belüftet ist, welche aus dem unteren Teil des Fußes kommen, der erhöht ist und eine Mehrzahl von äußeren Zugängen (8) aufweist.

4. Elektrischer Verdampfer nach Anspruch 3, **dadurch gekennzeichnet, dass** er ein in besagtem Fuß enthaltenes Gebläse (32) umfasst.

5. Elektrischer Verdampfer nach Anspruch 1, **dadurch gekennzeichnet, dass** die besagten Hohlkörper metallisch sind, mit zylindrischen Ausnehmungen, die eine vertikale Achse aufweisen, und in einem nach unten gerichteten Bereich mittels eines Widerstands (16) beheizt werden, der mit Hilfe eines Schalters (17) aktiviert werden kann und mit einem Thermostat ausgestattet ist.

6. Elektrischer Verdampfer nach Anspruch 1, **dadurch gekennzeichnet, dass** die besagten Hohlkörper am Kopf der Konstruktion befestigt sind, so dass eine Umfangsöffnung (29) bleibt, die es gestattet, dass die Belüftungsluft äußerlich über sie strömt, wobei ein aufsteigender Strom erzeugt wird, der die freigesetzten Dämpfe vermischt.

7. Elektrischer Verdampfer nach Anspruch 1, **dadurch gekennzeichnet, dass** der besagte Kopf durch eine abnehmbare Haube (27) geschlossen ist, um die Dämpfe zu schützen und zu vermischen, und mit unteren Öffnungen (29) für das Einströmen von Luft und mit oberen Öffnungen (28) für den Austritt der Dämpfe versehen ist.

8. Elektrischer Verdampfer nach Anspruch 1, **dadurch gekennzeichnet**, das Zeitsteuerungseinrichtungen zum Einstellen der Aktivierung und Deaktivierung der Heizwiderstände vorgesehen sind.

9. Elektrischer Verdampfer nach Anspruch 1, **dadurch gekennzeichnet, dass** die Temperaturen in drei Heizkörpern zwischen 90° und 110° Celsius zum Verdampfen von Propolisharz, zwischen 90° und 120° Celsius zum Verdampfen von Weihrauch bzw. zwischen 90° und 130° Celsius zum Verdampfen von Myrrhe liegen.

## Revendications

1. Évaporateur électrique multicorps pour résines,
**caractérisé en ce qu'**il comprend une structure de support (2) pour une pluralité de corps creux (13, 14, 15) électriquement chauffés, chacun d'entre eux étant conçu pour accueillir une ampoule (25) contenant un produit de résine à vaporiser, ladite structure contenant des moyens électriques et électroniques (12) d'alimentation et de commande permettant de vaporiser différents produits de résines à différentes températures, un dôme perforé (27) étant logé au-dessus des corps de chauffage et étant conçu pour protéger ceux-ci et pour mélanger les vapeurs générées.

2. Évaporateur électrique multicorps selon la revendication 1, **caractérisé en ce que** ladite structure de support est constituée d'un piétement (2) et d'une tête (3), tous deux étant creux.

3. Évaporateur électrique selon la revendication 2, **caractérisé en ce qu'**un transformateur réducteur (4) est contenu dans ledit piétement, dans une chambre (10) extérieurement ventilée par des flux d'air arrivant de la partie inférieure du piétement qui est surélevée et comporte plusieurs passages d'accès (8).

4. Évaporateur électrique selon la revendication 3, **caractérisé en ce qu'**il comprend un ventilateur (32) contenu dans ledit piétement.

5. Évaporateur électrique selon la revendication 1, **caractérisé en ce que** lesdits corps creux sont métalliques, avec des cavités cylindriques ayant un axe vertical, et sont chauffés dans une partie basse par une résistance (16) qui peut être activée au moyen d'un interrupteur (17), et qui est équipée d'un thermostat.

6. Évaporateur électrique selon la revendication 1, **caractérisé en ce que** lesdits corps creux sont fixés dans la tête de ladite structure de manière à garder une ouverture périphérique (29), qui permet audit air de ventilation de circuler extérieurement par-dessus ceux-ci, produisant ainsi un flux ascendant qui mélange les vapeurs dégagées.

7. Évaporateur électrique selon la revendication 1, **caractérisé en ce que** ladite tête est fermée par un dôme détachable (27) pour protéger et mélanger les vapeurs, et est pourvue d'ouvertures inférieures (29) permettant l'introduction d'air, et d'ouvertures supérieures (28) permettant la sortie des vapeurs.

8. Évaporateur électrique selon la revendication 1, **caractérisé en ce que** des moyens de minuterie sont prévus pour ajuster l'activation et la désactivation des résistances de chauffage.

9. Évaporateur électrique selon la revendication 1, **caractérisé en ce que** les températures dans trois corps de chauffage sont respectivement entre 90° et 110° Celsius pour vaporiser de la propolis, entre 90° et 120° Celsius pour vaporiser de l'encens, et entre 90° et 130° Celsius pour vaporiser de la myrrhe.
